Europäisches Patentamt

European Patent Office

Office européen des brevets

(11) **EP 0 387 693 B1**

(19)

(12) **EUROPÄISCHE PATENTSCHRIFT**

(45) Veröffentlichungstag und Bekanntmachung des Hinweises auf die Patenterteilung:
**25.09.1996 Patentblatt 1996/39**

(51) Int. Cl.$^6$: **A61M 37/00**

(21) Anmeldenummer: **90104406.5**

(22) Anmeldetag: **08.03.1990**

(54) **Transdermales System mit gestufter Wirkstoffabgabe und Verwendung für die lokale oder systemische, kosmetische Wirkstoffverabreichung**

Transdermal system with stepwise drug release, and its use in local or systemic cosmetic administration

Système transdermique de libération de médicament par paliers et son utilisation pour l'administration locale ou systémique de produits cosmétiques

(84) Benannte Vertragsstaaten:
**AT BE CH DE DK ES FR GB GR IT LI LU NL SE**

(30) Priorität: **15.03.1989 DE 3908431**

(43) Veröffentlichungstag der Anmeldung:
**19.09.1990 Patentblatt 1990/38**

(73) Patentinhaber: **LTS LOHMANN Therapie-Systeme GmbH**
**56567 Neuwied (DE)**

(72) Erfinder: **Müller, Walter, Dr.-Dipl.-Chem.**
**D-5450 Neuwied 1 (DE)**

(74) Vertreter: **Flaccus, Rolf-Dieter, Dr.**
**Patentanwalt**
**Sperlingsweg 32**
**50389 Wesseling (DE)**

(56) Entgegenhaltungen:
**EP-A- 0 113 562          EP-A- 0 227 252**
**EP-A- 0 242 827          EP-A- 0 249 343**
**US-A- 4 781 924**

**Beschreibung**

Die Erfindung betrifft ein wirkstoffhaltiges transdermales System zur gesteuerten abgestuften Verabreichung von Wirkstoffen an die Haut mit hoher Initialdosis und niedrigerer Erhaltungsdosis, bestehend aus einer der Haut abgewandten wirkstoffundurchlässigen Rückschicht, einem Wirkstoffreservoir mit einem Wirkstoff, einer haftklebenden Fixiereinrichtung für das System auf der Haut, einer gegebenenfalls die Flächen des Systems zur Haut hin abdeckenden ablösbaren Schutzschicht und einer im Abstand parallel zur Freigabefläche integrierten Membran, die im Wirkstoffreservoir enthalten ist.

Transdermale therapeutische Systeme haben sich mittlerweile ihren festen Platz in der Behandlung der verschiedensten Krankheiten erorbert. Ihr Hauptvorteil liegt darin, daß unter Umgehung der primären Leberpassage, die bei oral verabreichten Wirkstoffen gezwungenermaßen stattfindet, der Wirkstoff nach Permeation durch die Haut direkt systemisch zur Wirkung kommt und daß durch eine geeignete Auslegung des Systems sehr konstante Plasmaspiegel erreicht werden können. Dies ist besonders wichtig bei Wirkstoffen, die eine kurze Halbwertszeit besitzen und deshalb eine konstante Neuzufuhr an Wirkstoff notwendig machen.

Da das System extern appliziert wird, kann es ohne Wechsel sehr lange - bei einigen im Handel erhältlichen Systemen bis zu einer Woche - auf diese Weise seine ihm zugedachte Funktion erfüllen. Dies ist mit oralen Systemen schlechterdings unmöglich, da sie durch die Verdauungstätigkeit nach längstens einem Tag den Organismus verlassen haben.

Solche transdermalen Systeme bestehen überlicherweise aus einer für den Wirkstoff undurchlässigen Rückschicht, einem Wirkstoffreservoir, einer Fixiereinrichtung zur Verankerung des Systems auf der Haut und einer wiederentfernbaren Schutzfolie für die Hautseite des Systems. Eine bevorzugte Ausführung der Fixiereinrichtung besteht darin, daß die Hautseite des Systems zumindest teilweise selbstklebend ausgerüstet ist.

Das Reservoir kann in solchen Systemen die Form eines Beutels haben, der den flüssigen oder gelösten Wirkstoff enthält, oder ein mehr folienartiges Gebilde sein, das den Wirkstoff in einer polymerhaltigen Zubereitung enthält.

Die letzterwähnten Systeme werden auch Matrixsysteme genannt und alle folgenden Ausführungen beziehen sich auf Systeme dieser Art.

Besteht das Reservoir eines solchen Matrixsystems aus einer einzigen homogenen Schicht und liegen keine weiteren die Wirkstoffabgabe steuernden Schichten zwischen der nach Applikation der Haut zugewandten Seite des Reservoirs und der Haut selbst, so steuert das System die Wirkstoffabgabe, wenn die Matrix an Wirkstoff übersättigt ist nach Gleichung 1 bzw. wenn dies nicht der Fall ist nach Gleichung 2.

$$Q = ( D_{DR} * [C_{DR} - C_{SDR}] * C * t )^{1/2} \quad \text{Gl. 1}$$

$$Q = 2 * C_{DR} * ( D_{DR} * t / 3{,}14 )^{1/2} \quad \text{Gl. 2}$$

Q : zur Zeit t freigesetzte Menge Wirkstoff
t : Zeit
$D_{DR}$ : Diffusionskoeffizient des Wirkstoffs in der Matrix
$C_{DR}$ : Konzentration des Wirkstoffs in dem Reservoir
$C_{SDR}$ : Sättigungslöslichkeit des Wirkstoffs im Reservoir

Da Q direkt proportional der Quadratwurzel aus der Zeit ist, nennt man diese Gleichungen auch das Wurzel-t-Gesetz. Die Wirkstoffabgabe ist bei diesen Systemen nicht konstant und nimmt mit der Zeit schnell ab.

Ist eine konstantere Wirkstoffabgabe erwünscht, kann dies durch die Verwendung von sogenannten Steuermembranen erreicht werden.

Ein solches System besteht aus einer Rückschicht, dem Wirkstoffreservoir, der Steuermembran, einer Haftklebeschicht zur Befestigung des Systems auf der Haut und einer wiederentfernbaren Schutzfolie.

$$Q = Co * e^{-D / l * t} \quad \text{Gl. 3}$$

Co = Anfangskonzentration des Wirkstoffs
D = Diffusionskoeffizient des Wirkstoffs in der Membran
l = Dicke der Membran

Bei einigen Wirkstoffgruppen können jedoch konstante Abgaberaten und konstante Plasmaspiegel eher unerwünscht sein. Dazu gehören z.B. den Blutdruck und Kreislauf beeinflussende Mittel, Beruhigungs- und Schlafmittel, Psychopharmaka, Schmerzmittel, Wirkstoffe gegen Angina Pectoris, Antiasthmatika und die Suchtentwöhnung erleichternde Stoffe wie z.B. Nicotin. Bei diesen Substanzen ist es von Vorteil, wenn an den Bedarf angepasste Plasmaspiegel erreicht werden könnten.

Einige Wirkstoffe werden zudem nicht ständig verabreicht sondern nur bei Bedarf in möglicherweise sehr großen zeitlichen Abständen. Eine solche Substanz, die schon als transdermales System auf dem Markt ist, ist z.B. Scopolamin gegen Reisebeschwerden.

Andere Wirkstoffe, für die eine solche gelegentliche Anwendung denkbar ist, sind z.B. Schmerzmittel, Psychopharmaka, Beruhigungsmittel, Schlafmittel oder Appetitzügler.

Bei der transdermalen Applikation von solchen Substanzen muß das transdermale System dabei einen über den Applikationszeitraum variablen Wirkstofflux durch die Haut bewirken, wobei eine Initialdosis für einen raschen Wirkungseintritt sorgt und eine Erhaltungsdosis für eine genügend lange Konstanz oder vorprogrammiertes Abfallen der Plasmaspiegel.

Ein transdermales therapeutisches System zur Lösung dieses Problems ist schon im EP-A 0 227 252 beschrieben worden. Dort wird der Wirkstoff in einem Reservoir nur mit soviel Penetrationsbeschleuniger in Kontakt gebracht, daß die beschleunigte Penetration nur während einer definierten Anfangsphase der Applikation aufrechterhalten wird. Nachteilig ist hier, daß für jeden Wirkstoff ein geeigneter Penetrationsbeschleuniger gefunden werden muß.

Eine andere Problemlösung wird in der DE-OS 36 42 931 vorgeschlagen. Dort sind zumindest zwei nebeneinanderliegende und voneinander getrennte Kammern des Pflasters mit unterschiedlichen Wirkstoffkonzentrationen versehen, so daß in der ersten Phase der Applikation die Freigabe des Wirkstoffs aus allen Kammern eine hohe Initialdosis darstellt, während nach Entleerung der Kammern mit niedriger Wirkstoffkonzentration nur noch die Kammern mit höherer Wirkstoffkonzentration zur Freigabe beitragen und somit eine niedrigere Erhaltungsdosis bewirken. Dieses System ist schon von der Kammerkonstruktion her aufwendig und bedarf bezüglich der verschiedenen Konzentrationseinstellungen in den Kammern besonderer Maßnahmen.

Im EP-A-0 249 343 wird ein transdermales, therapeutisches System beschrieben, das mindestens eine im Abstand parallel zur Freigabefläche im Wirkstoffreservoir integrierte Membran aufweist. Diese Membran steuert durch ihre Permeabilität die Freigabe des Wirkstoffes.

Es ist daher Aufgabe der Erfindung, ein Pflaster als therapeutisches System zur Verabreichung von Wirkstoff an die Haut mit einer abgestuften Wirkstoffabgabe bereitzustellen, das die zwingende Anwesenheit eines Penetrationsbeschleunigers vermeidet, über den bisherigen Stand der Technik hinaus zusätzliche Möglichkeiten bietet, die Wirkstoffabgabe zu steuern und in einfacher Weise herstellbar ist.

Die Aufgabe wird erfindungsgemäß überraschenderweise dadurch gelöst, daß das wirkstoffhaltige Reservoir parallel zur Freigabefläche mindestens eine Membran enthält, die in ihrer flächigen Dimensionierung kleiner als die Freigabefläche ist.

Gegenstand der Erfindung ist somit ein transdermales System zur gesteuerten, abgestuften Verabreichung von Wirkstoffen an die Haut mit hoher Initialdosis und niedrigerer Erhaltungsdosis entsprechend den Merkmalen des Anspruchs 1

Unter einer Membran wird in diesem Zusammenhang ein flächenförmiges flexibles Gebilde verstanden, dessen Durchlässigkeit für Bestandteile des Wirkstoffreservoirs auch Null sein kann. Unter den Begriff Membran fällt also auch z.B. auch eine dünne Metallfolie. Üblicherweise übersteigt die Dicke einer solchen Membran selten 50 μ, jedoch sind auch dickere Membranen in speziellen Fällen nicht ausgeschlossen.

Üblich sind Membrandicken von 20 bis 100 μm.

Die Membran ist in das Reservoir integriert bzw. eingebettet.

Gemäß einer Ausführungsform ist die Membran für den oder die zur Freigabe bestimmten Wirkstoffe undurchlässig. Nach einer weiteren Ausführungsform ist die Membran für den oder die Wirkstoffe begrenzt durchlässig. Erfindungsgemäß können auch zwei Membranen kombiniert werden, die eine unterschiedliche Permeabilität für die zur Abgabe bestimmten Stoffe besitzen, von denen wenigstens eine eine kleinere Fläche als die der Freigabefläche des Systems hat. Für diesen Fall ist es vorteilhaft, daß die Membran, die flächenmäßig kleiner als die Freigabefläche des Systems ist, für den oder die zur Abgabe bestimmten Stoffe undurchlässig und in das Reservoir integriert ist.

Der oder die Wirkstoffe können im Reservoir entweder in einer die Sättigungskonzentration nicht übersteigenden oder in einer die Sättigungskonzentration übersteigenden Konzentration vorhanden sein.

Das Reservoir selbst kann wiederum aus verschiedenen Schichten unterschiedlicher Zusammensetzung bestehen.

Für alle Komponenten eines solchen Systems können prinzipiell die gleichen Materialien Verwendung finden, die für herkömmliche Systeme beschrieben sind. Diese Materialien sind dem Fachmann bekannt.

Die Rückschicht kann aus flexiblem oder nicht flexiblem Material bestehen und ein- oder mehrschichtig aufgebaut sein. Substanzen, die zu ihrer Herstellung verwendet werden können, sind polymere Substanzen, wie z.B. Polyethylen, Polypropylen, Polyethylenterephthalat, Polyurethan oder Polyamid. Als weitere Materialien können auch Metallfolien wie z.B. Aluminiumfolie, allein oder mit einem polymeren Substrat beschichtet, angewandt werden. Es können auch textile Flächengebilde verwendet werden, wenn die Bestandteile des Reservoirs dieses aufgrund ihrer physikalischen Daten nicht über die Gasphase verlassen können.

Für die wiederentfernbare Schutzfolie können im Prinzip die gleichen Materialien verwendet, werden, jedoch muß sie zusätzlich abhäsiv ausgerüstet sein. Die abhäsive Ausrüstung kann durch eine spezielle Silikonisierung erreicht werden.

Das Reservoir bzw. die Schichten des Reservoirs bestehen aus einer Polymermatrix und dem oder den Wirkstoffen, wobei die Polymermatrix eine solche Eigenklebrigkeit besitzt, daß der Zusammenhalt bei mehrschichtigem Aufbau gewährleistet ist. Das Polymermaterial der Matrix kann z.B. aufgebaut sein auf Polymeren wie Kautschuk, kautschukähnlichen synthetischen Homo-, Co- oder Blockpolymeren, Polyacrylsäureestern und deren Copolymerisate, Polyurethane, Copolymere des Ethylens und Polysiloxanen. Grundsätzlich kommen alle Polymere in Frage, die bei der Herstellung von Haftklebern eingesetzt werden und physiologisch unbedenklich sind. Es können noch Zusätze verwandt werden, deren Natur von dem eingesetzten Polymer und dem oder den Wirkstoff(en) abhängt. Nach ihrer Funktion lassen sie sich einteilen in Weichmacher, Klebrigmacher, Resorptionsvermittler, Stabilisatoren oder Füllstoffe. Die hierfür in Frage kommenden, physiologisch unbedenklichen Substanzen sind dem Fachmann bekannt.

Für die Membranen können alle physiologisch unbedenklichen folienartige Materialien eingesetzt werden, die für den Anwendungszweck die geeignete Durchlässigkeit für den oder die Wirkstoffe bzw. Hilfsstoffe aufweisen. Besonders geeignet sind Membranen auf der Basis von Polyethylen, Polyamid, Ethylen-Vinylacetat-Copolymeren und Polysiloxanen.

Die Erfindung wird nachfolgend anhand der Figuren näher erläutert. Dabei zeigt:

Figur 1     einen Schnitt durch ein System mit einer Steuermembran

Figur 3     zeigt eine Ansicht der Matrix mit in die Matrix integrierter Membran

Figur 4     verschiedene Ausführungsformen der Membran,

Figur 5a    einen Schnitt durch eine Ausführungsform der Erfindung, die eine Kombination einer undurchlässigen Membran mit einer Membran größerer Permeabilität aufweist,

Figur 5b    eine Draufsicht auf die Ausführungsform von Figur 5a

Figur 6     in grafischer Darstellung das Freisetzungsverhalten einer Ausführungsform mit in das Reservoir integrierter, für den Wirkstoff undurchlässiger Membran einer Form, wie sie in Figur 4 dargestellt ist, wobei die kumulierte freigesetzte Menge Wirkstoff als Funktion der Zeit dargestellt ist.

Figur 7     das Freisetzungsverhalten des gleichen Systems wie in Figur 6, jedoch in grafischer Darstellung die Abgaberate des Wirkstoffs pro System und Stunde als Funktion der Zeit dargestellt,

Figur 8     in grafischer Darstellung das Freisetzungsverhalten eines erfindungsgemäßen Systems mit einer für den Wirkstoff begrenzt durchlässigen Membran, wobei die kumulative Freisetzung des Wirkstoffs über die Zeit dargestellt ist,

Figur 9     in grafischer Darstellung die Abgaberate des gleichen Systems wie in Figur 8 pro System und Stunde als Funktion der Zeit,

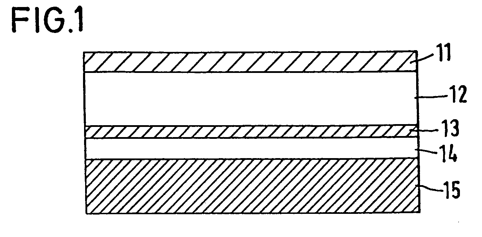

Das in Figur 1 beschriebene System besteht aus einer Rückschicht (11), dem Wirkstoffreservoir (12), der Steuermembran (13), einer Haftklebeschicht zur Befestigung des Systems auf der Haut (14) und einer wiederentfernbaren Schutzfolie (15).

Erfindungsgemäß hat die Haftklebeschicht (14) die gleiche Formulierung wie das Reservoir (12), so daß im Prinzip die Membran in das Reservoir integriert ist und man sich deshalb das Reservoir aus zwei Teilen aufgebaut denken kann.

Durch die Membran erreicht man, wenn der Wirkstoff in übersättigter Konzentration im Reservoir vorliegt, eine Freisetzung nach einer Kinetik 0. Ordnung, d.h. eine konstante Freisetzung über den Applikationszeitraum, und wenn der Wirkstoff unterhalb dieser Konzentration vorliegt eine Freigabe nach einer Kinetik 1. Ordnung gemäß Gleichung 3.

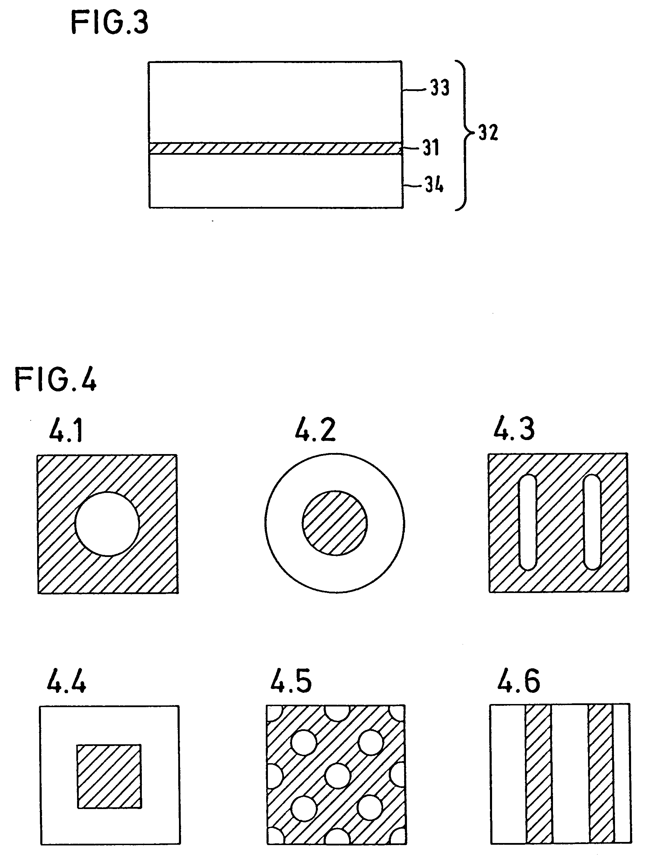

In Figur 3 ist eine Membran (31) in das Reservoir (32) integriert, das dadurch in zwei Hälften (33 und 34) unterteilt wird. Ist die Reservoirformulierung selbstklebend , so kann natürlich der selbstklebende Hautstrich entfallen. Bedingt dadurch, daß die Fläche der Membran immer kleiner als die Gesamtfläche des Reservoirs ist, haben auf der nicht von der Membran abgedeckten Fläche Reservoir und Haut, bzw. Reservoir und Haftkleberschicht bzw. die beiden Teile des Reservoirs direkten Kontakt miteinander.

Figur 4 zeigt einige Beispiele für die geometrische Gestaltung einer solchen Membran gemäß der Erfindung, wo entweder die schraffierten Flächen oder die nicht schraffierten Flächen Membranen sind.

Die erfindungsgemäßen Ausführungsformen nach Figuren 3 und 4 sind besonders geeignet für Systeme mit nur einer für den Wirkstoff undurchlässigen Membran, z.B wie sie in Figur 4.1 ausgeführt ist und entsprechend Figur 3 in das Reservoir integriert ist.

Zunächst verhält sich dieses System wie ein gewöhnliches Matrixsystem, d.h. über die gesamte Freigabefläche wird der Wirkstoff nach dem sogenannten Wurzel-t-Gesetz abgegeben. Wenn jedoch der Reservoirteil, der unterhalb der Membranfläche liegt, soweit entleert ist, daß die Verarmungszone die Membran erreicht hat, ändert sich sein Verhalten im Vergleich zu einem herkömmlichen Matrixsystem drastisch. Auf der in ihrer Größe der Membran entsprechenden Fläche geht die Wirkstoffabgabe sehr schnell zurück, während auf der nicht von der Membran bedeckten Teilfläche die Freisetzung nach dem Wurzel-t-Gesetz unvermindert anhält, bis die Verarmungszone die Rückschicht erreicht. Die zusätzliche Initialdosis stammt also von der unterhalb der Membran liegenden Fläche. Durch Änderungen der absoluten Flächengrößen und des Verhältnisses zwischen Membranfläche und Gesamtfläche des Reservoirs läßt sich die Größe der Initial- und Erhaltungsdosis in weiten Grenzen beeinflussen.

Ein solches Freisetzungsverhalten kann man natürlich auch dadurch erreichen, daß man dem Reservoir eine stufenförmige Geometrie gibt. Dies hat aber den Nachteil, daß ein solches System schwieriger zu fertigen ist, und daß aufgrund der plastischen Verformbarkeit der üblichen Reservoirformulierung ein solches System seine stufenförmige Gestalt nicht behält.

Besonders vorteilhaft ist die Ausführung 4.5, da durch die Vielzahl der sich in der Membran befindlichen Löcher keine Positionierungsprobleme auftreten.

Durch Änderung des Verhältnisses der Membranfläche zur Gesamtfläche und der Wahl von Membranen verschiedenen Permeabilität für den Wirkstoff, läßt sich, wie nachfolgend gezeigt, das Freisetzungsverhalten des Systems in weiten Grenzen beeinflussen. Insbesondere ist es möglich, sehr hohe Initialdosen zu verabreichen.

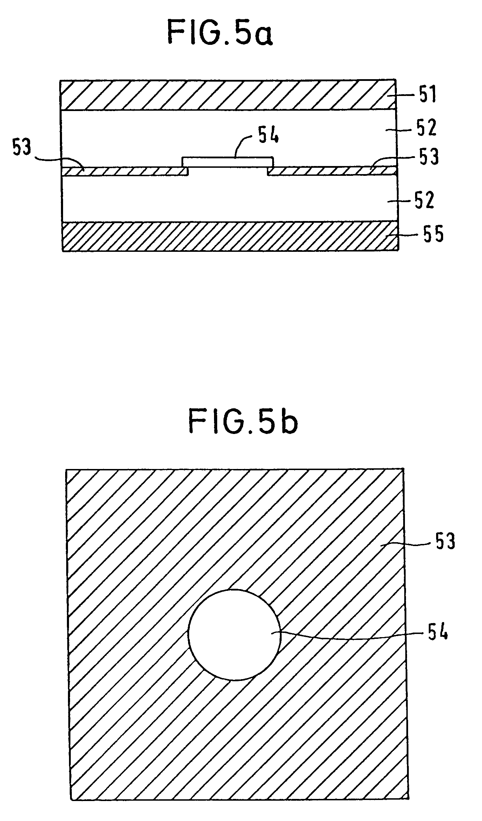

Figuren 5a und 5b zeigen im Schnitt und in Draufsicht eine Ausführungsform gemäß der Erfindung, die eine Kombination zweier verschiedener Membranen aufweist. Besonders sinnvoll ist z.B. die Kombination von einer Membran der Permeabilität "0" mit einer Membran größerer Permeabilität. Ein solches System ist in Figur 5a gezeigt. Es besteht aus der undurchlässigen Rückschicht (51), dem Reservoir (52), einer Membran mit der Permeabilität 0 für den oder die Wirkstoffe (53), einer Membran mit einer Permeabilität größer 0 für den oder die Wirkstoffe (54) und einer wiederentfernbaren Schutzschicht (55).

Die beiden Membranen sind in Figur 5b nochmals in der Aufsicht gezeigt. Die Membran mit der Permeabilität 0 muß natürlich flächenmäßig kleiner als sein als die Gesamtabgabefläche des Systems und begrenzt so die maximale Wirkstoffabgabe auf der ihrer Größe entsprechenden Teilfläche der Gesamtfreigabefläche, da der über ihr liegende Anteil Wirkstoff sie nicht passieren kann.

Die Membran mit der Permeabilität größer 0 führt auf der ihr entsprechenden Teilfläche der Gesamtabgabefläche zu einer Wirkstoffabgabe nach einer Kinetik nullter oder erster Ordnung.

Die beiden Membranen müssen innerhalb des Systems nicht gezwungenermaßen in der gleichen Ebene liegen. Ihre genaue Position richtet sich nach den jeweiligen Anforderungen und ist ein zusätzliches Mittel das gewünschte Freisetzungsverhalten zu erreichen.

Wenn die Membran mit der Permeabilität 0 näher der Freigabefläche liegt, kann die andere Membran, ohne daß sich das Freisetzungsverhalten ändert, flächenmäßig ebenso groß wie die Gesamtabgabefläche sein, da sie oberhalb der undurchlässigen Membran keinerlei Wirkung hervorruft.

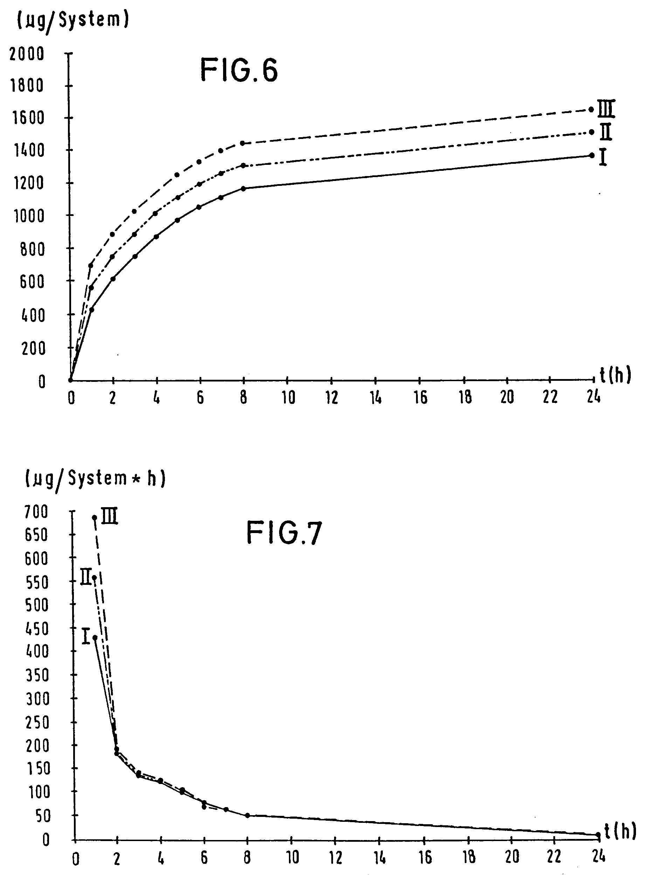

In Figur 6 und 7 ist das Freisetzungsverhalten solcher Systeme mit einer für den Wirkstoff undurchlässigen Membran am Beispiel von Scopolaminpflaster gezeigt. Für alle der nachfolgend beschriebenen Muster gilt, daß der Wirkstoffgehalt 450 $\mu$g/cm$^2$ und das Flächengewicht des selbstklebenden Reservoirs 12,5 mg/cm$^2$ beträgt.

In Figur 6 ist die kumulierte freigesetzte Menge Scopolamin als Funktion der Zeit dargestellt.

Kurve I entspricht einem normalen 2 cm$^2$ großen System ohne Membran und dient als Vergleich.

Kurve II entspricht einem insgesamt 3 cm$^2$ großen System, in dessen Reservoir eine undurchlässige Membran integriert ist. Die Membran hat eine Fläche von 1 cm$^2$ und teilt das Reservoir in eine Schicht mit einem Flächengewicht von 10,4 mg/cm$^2$ und eine mit einem Flächengewicht von 2,1 mg/cm$^2$.

Kurve III entspricht einem System mit der Gesamtfläche von 4 cm$^2$ und einer Membranfläche von 2 cm$^2$.

Es ist deutlich die insgesamt höhere Wirkstoffabgabe der Systeme mit Membran zu erkennen. Daß sich diese erhöhte Wirkstoffabgabe allerdings nur auf die Anfangsphase der Freisetzung beschränkt, ist besser in Figur 7 zu erkennen, bei der die Abgaberate pro System und Stunde als Funktion der Zeit, d.h. der Flux aufgetragen ist.

Dieses System ist also besonders gut geeignet, wenn man relativ hohe Initialdosen mit einer nicht unbedingt konstanten Erhaltungsdosis kombinieren will.

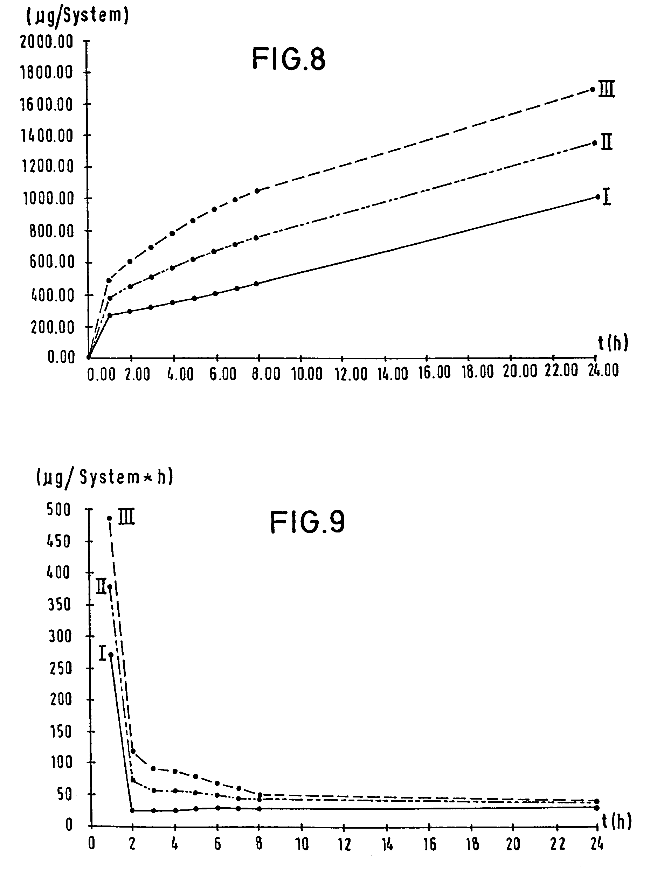

Figuren 8 und 9 zeigen eine erfindungsgemäße Ausführungsform mit einer für den Wirkstoff begrenzt durchlässigen Membran am Beispiel von Scopolaminpflaster und unter den gleichen Bedingungen wie bei Figuren 6 und 7 geschildert.

Die kumulative Freisetzung ist in Figur 8 und der Flux in Figur 9 dargestellt. Kurve I bzw. Flux I entspricht einem 2 cm$^2$ großen System, das eine Membran gleicher Größe enthält, (Vergleich) Kurve II bzw. Flux II entsprechen einem 2,5 cm$^2$ großen System mit einer 2 cm$^2$ großen Membran und Kurve 212 bzw. Flux III entsprechen einem 3 cm$^2$ großen System mit einer 2 cm$^2$ großen Membran.

Auch das System entsprechend Kurve 2 und Flux 2 kann eine gewisse Initialdosis abgeben. Diese Initialdosis entspricht einer Freisetzung nach dem Wurzel-t-Gesetz gemäß Gleichung 1 und 2, die stattfindet, bis die Verarmungszone des Wirkstoffs die Membran erreicht hat.

Die beiden anderen Systeme mit einer Membran, die flächenmäßig kleiner als die Freigabefläche des Systems sind, und die Erfindung veranschaulichen jedoch, wie einfach diese Initialdosis erhöht werden kann. In diesem Fall folgt auf die Initialdosis eine konstante Erhaltungsdosis, deren Höhe von der Permeabilität der Membran und der Membranfläche abhängt.

Herstellung der in den Figuren 6 bis 9 verwendeten erfindungsgemäßen Systeme (Muster)

| | |
|---|---|
| 230 g | Polyacrylharzkleber (50 % in Essigester) |
| 6 g | Scopolamin-Base |
| 10 g | Cetiol S |
| 50 g | Methanol |

wurden zusammengegeben und die Mischung homogenisiert. Mit dieser Mischung wurden eine 100 μ dicke silikonisierte Polyesterfolie 400 μ (Film I) und 100 μ dick (Film II) beschichtet und die Filme bei 50°C 15 Minuten getrocknet. Film I hatte nach dem Trocknen ein Flächengewicht von 103 g/m$^2$ und Film II eines von 21 g/m$^2$.

Auf Film II wurde die Membran mit kreisförmigen Aussparungen entsprechender Größe aufkaschiert und darauf wiederum Film I. Die silikonisierte Polyesterfolie von Film I wurde abgezogen und durch eine 15 μ dicke nichtsilikonisierte Folie ersetzt.

Danach wurden die einzelnen Muster so ausgestanzt, daß sich die entsprechende Gesamtfläche ergab und die Aussparung zentral zu liegen kam.

Durchführung der in vitro-Freisetzung

Die Freisetzung wurde bei 32°C nach der Paddle-over-disk Methode mittels 50 ml physiologischer Kochsalzlösung durchgeführt. Zur Probennahme wurde das gesamte Freisetzungsmedium komplett gewechselt und der Gehalt mit einer HPLC-Methode gemessen.

## Patentansprüche

**Patentansprüche für folgende Vertragsstaaten : AT, BE, CH, LI, DE, DK, FR, GB, IT, LU, NL, SE**

1. Wirkstoffhaltiges transdermales System zur gesteuerten abgestuften Verabreichung von Wirkstoffen an die Haut mit hoher Initialdosis und niedrigerer Erhaltungsdosis, bestehend aus einer der Haut abgewandten wirkstoffundurchlässigen Rückschicht (11, 21), einem Wirkstoffreservoir (12, 14) mit einem Wirkstoff, einer haftklebenden Fixiereinrichtung für das System auf der Haut, einer gegebenenfalls die Flächen des Systems zur Haut hin abdeckenden, wiederablösbaren Schutzschicht (15, 25) und mindestens einer im Abstand parallel zur Freigabefläche integrierten Membran (13, 23), die im Wirkstoffreservoir (12, 14) enthalten ist, dadurch gekennzeichnet, daß die Permeabilität der Membran für den/die zur Freigabe bestimmten Wirkstoffe Null ist oder größer als Null ist, und daß die Membranfläche kleiner als die Abgabefläche des Systems ist.

2. Transdermales System nach Anspruch 1, dadurch gekennzeichnet, daß es mindestens 2 Membranen (53, 54) enthält, die eine unterschiedliche Permeabilität für die zur Freigabe bestimmten Wirkstoffe besitzen, und von denen wenigstens eine (53) flächenmäßig kleiner als die Freigabefläche des Systems ist.

3. Transdermales System gemäß Anspruch 4, dadurch gekennzeichnet, daß die Membran (53), die flächenmäßig kleiner als die Freigabefläche des Systems ist, für die zur Freigabe bestimmten Wirkstoffe die Permeabilität 0 aufweist.

4. Transdermales System gemäß einem der Ansprüche 1-5, dadurch gekennzeichnet, daß das Reservoir (12, 14, 22, 24, 33, 34, 52) den/die Wirkstoff(e) in einer die Sättigungskonzentration nicht übersteigenden Konzentration enthält.

5. Transdermales System gemäß einem der Ansprüche 1-5, dadurch gekennzeichnet, daß das Reservoir (12, 14, 22, 24, 33, 34, 52) den/die Wirkstoff(e) in einer die Sättigungskonzentration übersteigenden Konzentration enthält.

6. Transdermales System gemäß einem der Ansprüche 1-7, dadurch gekennzeichnet, daß die wirkstoffhaltigen Teile des Reservoirs aus mehreren Schichten unterschiedlicher Zusammensetzung bestehen.

7. Verwendung des transdermalen Systems nach einem der Ansprüche 1-9 für die lokale oder systemische dermale Wirkstoffverabreichung in der Kosmetik.

**Patentansprüche für folgende Vertragsstaaten : ES, GR**

1. Verfahren zur Herstellung eines transdermalen Systems zur gesteuerten, abgestuften Verabreichung eines zur Freigabe bestimmten Wirkstoffes an die Haut mit hoher Initialdosis und niedrigerer Erhaltungsdosis, bestehend aus einer der Haut abgewandten wirkstoffundurchlässigen Rückschicht, einem Wirkstoffreservoir und einer haftklebenden Fixiereinrichtung für das System auf der Haut, einer gegebenenfalls die Flächen des System zur Haut hin abdeckenden, wiederablösbaren Schutzschicht, und mindestens einer im Abstand parallel zur Freigabefläche integrierte Membran (13, 23), die im Wirkstoffreservoir (12, 14) eingearbeitet wird, dadurch gekennzeichnet, daß die Permeabilität der Membran für den/die zur Freigabe bestimmten Wirkstoffe Null ist oder größer als Null ist, und daß die Fläche der Membran (13, 23) kleiner als die Freigabefläche des Wirkstoffreservoirs (12, 14) bemessen wird.

2. Verfahren nach Anspruch 1, dadurch gekennzeichnet, daß mindestens zwei Membranen verwendet werden, die eine unterschiedliche Permeabilität für die zur Freigabe bestimmten Wirkstoffe besitzen, von denen wenigstens eine flächenmäßig kleiner als die Freigabefläche des Systems ist.

3. Verfahren nach Anspruch 4, dadurch gekennzeichnet, daß die Membran, welche flächenmäßig kleiner als die Freigabefläche des Systems ist, für die zur Freigabe bestimmten Wirkstoffe undurchlässig ist.

4. Verfahren nach einem der Ansprüche 1-5, <u>dadurch gekennzeichnet,</u> daß das verwendete Reservoir den/die Wirkstoff(e) in einer die Sättigungskonzentration nicht übersteigenden Konzentration enthält.

5. Verfahren nach einem der vorangegangenen Ansprüche, <u>dadurch gekennzeichnet,</u> daß das verwendete Reservoir den/die zur Freigabe bestimmten Wirkstoff(e) in einer die Sättigungskonzentration übersteigenden Konzentration enthält.

6. Verfahren nach einem der vorangegangenen Ansprüche, <u>dadurch gekennzeichnet,</u> daß die wirkstoffhaltigen Teile des verwendeten Reservoirs aus mehreren Schichten unterschiedlicher Zusammensetzung bestehen-.

7. Verwendung des nach Ansprüchen 1-8 hergestellten transdermalen Systems für die lokale oder systemische dermale Wirkstoffverabreichung in der Kosmetik.

## Claims

### Claims for the following Contracting States : AT, BE, CH, LI, DE, DK, FR, GB, IT, LU, NL, SE

1. Active substance-containing transdermal system for the controlled, stepwise administration of active substances to the skin, having a high initial dose and a lower maintenance dose, consisting of an active substance-impermeable backing layer (11) averted from the skin, an active substance reservoir (12, 14) with an active substance, a pressure-sensitive fixation device for securing the system on the skin, a removable protective layer (15) optionally covering the surfaces of the system towards the skin, and at least one membrane (13) integrated parallelly in distance to the release surface, which membrane (13) is contained in the active substance reservoir (12, 14), characterized in that the permeability of the membrane for the active substance(s) to be released is equal to zero or greater than zero, and that the membrane surface is smaller than the release surface of the system.

2. The transdermal system according to claim 1 characterized in that it contains at least 2 membranes (53, 54) which have different permeabilities for the active substances to be released, and of which at least one (53) is smaller in area than the release surface of the system.

3. The transdermal system according to claim 2 characterized in that the membrane (53) which is smaller in area than the release surface of the system has the permeability of 0 for the active substances to be released.

4. The transdermal system according to one of claims 1 - 3, characterized in that the reservoir (12, 14, 33, 34, 52) contains the active substance(s) in a concentration not exceeding the saturation concentration.

5. The transdermal system according to one of claims 1 - 3, characterized in that the reservoir (12, 14, 33, 34, 52) contains the active substance(s) in a concentration exceeding the saturation concentration.

6. The transdermal system according to one of claim 1 - 5, characterized in that the active substance-containing parts of the reservoir consist of several layers of different composition.

7. The use of the transdermal system according to one of claims 1 - 6 for local or systemic dermal active substance administration in cosmetics.

### Claims for the following Contracting States : ES, GR

1. A process for producing a transdermal system for the controlled, stepwise administration of an active substance which is to be released, to the skin, having a high initial dose and a lower maintenance dose, consisting of an active substance-impermeable backing layer averted from the skin, an active substance reservoir, and a pressure-sensitive fixation device for securing the system on the skin, a removable protective layer optionally covering the surfaces of the system towards the skin, and at least one membrane (13) integrated parallelly in distance to the release surface, which membrane (13) is incorporated in the active substance reservoir (12, 14), characterized in that the permeability of the membrane for the active substance(s) to be released is equal to zero or greater than zero, and that the surface of the membrane (13) is dimensioned smaller than the release surface of the active substance reservoir (12, 14).

2. The process according to claim 1 characterized in that at least 2 membranes are used which have different permeabilities for the active substances to be released, and of which at least one is smaller in area than the release surface of the system.

3. The process according to claim 2 characterized in that the membrane which is smaller in area than the release surface of the system is impermeable to the active substances to be released.

4. The process according to one of claims 1 - 3, characterized in that the reservoir which is used contains the active substance(s) in a concentration not exceeding the saturation concentration.

5. The process according to one of the preceding claims, characterized in that the reservoir which is used contains the active subtance(s) in a concentration exceeding the saturation concentration.

6. The process acording to one of the preceding claims, characterized in that the active substance-containing parts of the reservoir consist of several layers of different composition.

7. The use of the transdermal system according to one of claims 1 - 6 for local or systemic dermal active substance administration in cosmetics.

**Revendications**

**Revendications pour les Etats contractants suivants : AT, BE, CH, LI, DE, DK, FR, GB, IT, LU, NL, SE**

1. Système transdermique contenant un principe actif pour l'administration échelonnée et réglée de principes actifs à la peau avec dose initiale élevée et dose d'entretien plus faible, se composant d'une couche dorsale (11, 21) imperméable au principe actif à l'opposé de la peau, d'un réservoir de principe actif (12, 14) avec un principe actif, d'un dispositif de fixation autocollant pour le système sur la peau, d'une couche protectrice (15, 25) amovible recouvrant éventuellement la surface du système en direction de la peau, et d'au moins une membrane (13, 23) intégrée parallèle à distance par rapport à la surface de libération, laquelle membrane est contenue dans le réservoir de principe actif (12, 14), caractérisé en ce que la perméabilité de la membrane au/aux principes actifs destinés à être libérés est égale à zéro ou supérieure à zéro et en ce que la surface de la membrane est plus petite que la surface de libération du système.

2. Système transdermique suivant la revendication 1, caractérisé en ce qu'il comprend au moins deux membranes (53, 54) qui possèdent une perméabilité différente aux principes actifs destinés à être libérés et dont l'une au moins (53) est de surface plus petite que la surface de libération du système.

3. Système transdermique suivant la revendication 4, caractérisé en ce que la membrane (53) de surface plus petite que la surface de libération du système présente la perméabilité 0 pour les principes actifs destinés à être libérés.

4. Système transdermique suivant l'une quelconque des revendications 1 à 5, caractérisé en ce que le réservoir (12, 14, 22, 24, 33, 34, 52) contient le ou les principes actifs en une concentration ne dépassant pas la concentration de saturation.

5. Système transdermique suivant l'une quelconque des revendications 1 à 5, caractérisé en ce que le réservoir (12, 14, 22, 24, 33, 34, 52) contient le ou les principes actifs en une concentration supérieure à la concentration de saturation.

6. Système transdermique suivant l'une quelconque des revendications 1 à 7, caractérisé en ce que les parties du réservoir qui contiennent le principe actif sont constituées de plusieurs couches de composition différente.

7. Utilisation du système transdermique selon l'une quelconque des revendications 1 à 9, pour l'administration du principe actif par voie dermique locale ou systémique en cosmétique.

**Revendications pour les Etats contractants suivants : ES, GR**

1. Procédé de préparation d'un système transdermique pour l'administration échelonnée et réglée d'un principe actif destiné à être libéré à la peau avec dose initiale élevée et dose d'entretien plus faible, se composant d'une couche dorsale imperméable au principe actif à l'opposé de la peau, d'un réservoir de principe actif et d'un dispositif de fixation autocollant pour le système sur la peau, d'une couche de protection amovible, recouvrant éventuellement la surface du système en direction de la peau, et d'au moins une membrane (13, 23) intégrée parallèle à distance à la surface de libération, qui est incorporée au réservoir de principe actif (12, 14), caractérisé en ce que la perméabilité de la membrane au/aux principes actifs destinés à être libérés est égale à zéro ou est supérieure à zéro et en ce que la surface de la membrane (13, 23) est mesurée de manière à être plus petite que la surface de libération du principe actif (12, 14).

2. Procédé suivant la revendication 1, caractérisé en ce que l'on utilise au moins deux membranes qui possèdent une perméabilité différente aux principes actifs destinés à être libérés et dont l'une au moins est de surface plus petite que la surface de libération du système.

3. Procédé suivant la revendication 4, caractérisé en ce que la membrane qui est de surface plus petite que la surface de libération du système est imperméable aux principes actifs destinés à être libérés.

4. Procédé suivant l'une quelconque des revendications 1 à 5, caractérisé en ce que le réservoir utilisé contient le ou les principes actifs en une concentration ne dépassant pas la concentration de saturation.

5. Procédé suivant l'une quelconque des revendications précédentes, caractérisé en ce que le réservoir utilisé contient le ou les principes actifs destinés à être libérés en une concentration supérieure à la concentration de saturation.

6. Procédé suivant l'une quelconque des revendications précédentes, caractérisé en ce que les parties du réservoir qui contiennent le principe actif sont constituées de plusieurs couches de composition différente.

7. Utilisation du système transdermique préparé selon l'une quelconque des revendications 1 à 8, pour l'administration du principe actif par voie dermique locale ou systémique en cosmétique.

# FIG.1

## FIG.3

## FIG.4

### 4.1

### 4.2

### 4.3

### 4.4

### 4.5

### 4.6

# FIG.5a

# FIG.5b

FIG.6

FIG.7

FIG.8

FIG.9